# EUROPÄISCHE PATENTSCHRIFT

(11) **EP 3 458 024 B1**
(45) Veröffentlichungstag und Bekanntmachung des Hinweises auf die Patenterteilung: **13.01.2021**
(21) Anmeldenummer: 17722047.2
(22) Anmeldetag: 11.05.2017
(51) Int. Cl.: A61K 8/46, C11D 1/00, A61K 8/41, A61Q 5/02, A61Q 9/02, A61Q 19/10, C11D 1/12, C11D 1/28, C11D 1/37, C11D 3/04, C11D 1/04

(54) **WÄSSRIGE TENSID-ZUSAMMENSETZUNGEN**
AQUEOUS TENSIDE COMPOSITIONS
COMPOSITIONS DE TENSIOACTIFS AQUEUX

(30) Priorität: 18.05.2016 EP 16170204
(43) Veröffentlichungstag der Anmeldung: 27.03.2019
(73) Patentinhaber: BASF SE, 67056 Ludwigshafen am Rhein (DE)
(72) Erfinder: BRUNN, Claudia, 40589 Duesseldorf-Holthausen (DE); BEHLER, Ansgar, 40589 Düsseldorf-Holthausen (DE)
(74) Vertreter: BASF IP Association
(86) Internationale Anmeldenummer: PCT/EP2017/061324
(87) Internationale Veröffentlichungsnummer: WO 2017/198537

(56) Entgegenhaltungen:
- WO-A1-98/44907
- WO-A1-2016/030172
- FR-A1- 3 013 968
- DATABASE GNPD [Online] MINTEL; 1. Dezember 2005 (2005-12-01), Anonymous: "Hair and Body Shampoo", XP002762134, Database accession no. 3600935

## Beschreibung

### Gebiet der Erfindung

Die vorliegende Erfindung betrifft wässrige Tensid-Zusammensetzungen mit einem Gehalt an alpha-Sulfofettsäuredisalzen sowie Sulfosuccinate.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Shampoozusammensetzungen für Haut und Haar enthaltend die anionischen Tenside Disodium 2-Sulfolaurate (A) und Sodiumlaureth Sulfosuccinate (B) sind bereits auf dem Markt bekannt (DATABASE GNPD [Online]; MINTEL; 1. Dezember 2005 (2005-12-01), Anonymous: "Hair and Body Shampoo", Database accession no. 3600935.) Auch die marktüblichen Produkte STEPAN MILD LSB und STEFAN MILD, offenbart in einer kosmetischen wässrigen Reinigungs-Zusammensetzung in der französischen Patentanmeldung FR 3 013 968 A1, enthalten geringe Mengen an Disodium 2-Sulfolaurate. Die Anmelderin beschreibt in der Internationalen Anmeldung WO 2016/030172 A1 wässrige Tensid-Zusammensetzungen enthaltend ein oder mehrere alpha-Sulfofettsäuredisalze (A) in Kombination mit ein oder mehreren N-Acyl-Glutaminsäure-Verbindungen. Ebenfalls sind wässrige Zusammensetzungen mit alpha-Sulfofettsäuredisalzen aus der PCT-Anmeldung WO 98/44907 A1 bekannt.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können.

### Beschreibung der Erfindung

Die Aufgabe der vorliegenden Erfindung hat darin bestanden, wässrige Tensid-Zusammensetzungen bereitzustellen, die sich durch die im Folgenden genannten Eigenschaften auszeichnen:
- Gutes Schaumvermögen.
- Angenehme Sensorik des Schaumes.
- Gute Hautverträglichkeit.

Gegenstand der Erfindung sind zunächst wässrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Sulfosuccinate (B)** der allgemeinen Formel (II),

   R³¹-O-CO-CH(SO₃M¹⁶)-CH₂-COOM¹⁷ (II)

   wobei der Rest R³¹ einen linearen oder verzweigten Alkyl- oder Alkenylrest mit 6 bis 22 C-Atomen oder einen alkoxylierten linearen oder verzweigten Alkyl- oder Alkenylrest mit 6 bis 22 C-Atomen bedeutet und die Reste M¹⁶ und M¹⁷ - unabhängig voneinander - ausgewählt werden aus der Gruppe H, Li, Na, K, Ca/2, Mg/2, Ammonium und Alkanolamine,
- ein oder mehrere **Verbindungen (C)** der allgemeinen Formel (III)

   R⁴COOM⁵ (III)

   worin der Rest R⁴ einen linearen oder verzweigten Alkyl- oder Alkenylrest mit 7 bis 19 C-Atomen bedeutet und der Rest M⁵ ausgewählt wird aus der Gruppe H, Li, Na, K, Ca/2, Mg/2, Ammonium und Alkanolamin,
- ein oder mehrere **anorganische Salze der Schwefelsäure (D)** der allgemeinen Formel (IV)

   (M⁶)₂SO₄ (IV)

   enthalten, wobei M⁶ ausgewählt wird aus der Gruppe Li, Na, K, Ca/2, Mg/2, Ammonium und Alkanolamin,
- **Wasser,**
   wobei folgende Maßgabe gilt:
- Sofern die wässrigen Tensid-Zusammensetzungen ein oder mehrere Estersulfonate (E) der allgemeinen Formel (V),

   R²CH(SO₃M⁷)COOR³ (V)

   worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 90 Gew.-% oder mehr - vorliegen müssen.

Die erfindungsgemäßen wässrigen Tensid-Zusammensetzungen zeichnen sich durch folgende vorteilhafte Eigenschaften aus:
- Gutes Schaumvermögen und angenehme Sensorik des Schaumes. Hierzu sei angemerkt, dass insbesondere im Bereich der Kosmetik unter Schaumvermögen verschiedene Aspekte verstanden werden können, beispielsweise können sowohl Schaumvolumen, Schaumstabilität, Schaumelastizität, Wassergehalt des Schaumes als auch optische Merkmale des Schaumes wie beispielsweise die Porengröße zur Beurteilung des Schaumes herangezogen werden. Die erfindungsgemäßen Zusammensetzungen weisen ein großes Schaumvolumen während des Anschäumens auf. In der Praxis findet das Anschäumen in einem relativ kurzen Zeitraum statt (von wenigen Sekunden bis zu einer Minute). Typischerweise wird beim Anschäumen ein Duschgel oder ein Shampoo durch Reiben zwischen Händen, Haut und/oder Haaren verteilt und zum Schäumen gebracht. Im Labor kann das Anschäumverhalten einer wässrigen Tensidlösung z.B. dadurch beurteilt werden, dass man in einer vergleichbar kurzen Zeitspanne die Lösung durch Rühren, Schütteln, Pumpen, Durchperlen eines Gasstroms oder auf andere Weise in Bewegung versetzt. Eine subjektive Beurteilung der Schaumsensorik kann in einem Probandentest erfolgen. Hierbei können Aspekte wie beispielsweise Cremigkeit, Elastizität, Formbarkeit des Schaums beurteilt werden.
- Gute Haut- bzw. Schleimhaut-Verträglichkeit. Diese kann durch den Fachmann bekannte invitro Methode (bspw. RBC oder HET-CAM) als auch Probandentests (bspw. Patchtest) nachgewiesen werden.
- Hervorragende Pflegeleistung an Haut und Haar. Diese kann beispielsweise im Probandentest anhand des subjektiven Hautgefühls (Glätte, Trockenheit etc.) oder Haptik und Griff des behandelten Haares beurteilt werden. Es können ebenso mechanische Messmethoden wie bspw. Kämmarbeit am Haar herangezogen werden.
- Gute Lagerstabilität. Diese ist dann gegeben, wenn die wässrigen Zusammensetzungen über einen Zeitraum von mehreren Wochen keine sichtbaren (z.B. Austrübung, Verfärbung, Phasentrennung) oder messbaren (z.B. pH-Wert, Viskosität, Aktivsubstanzgehalt) Veränderungen erfahren.
- Gute Anwendbarkeit und Verarbeitbarkeit. Die Zusammensetzungen lassen sich bei Einbringen in Wasser schnell und ohne Wärmezufuhr verlösen.
- Gute Klarlöslichkeit und Transparenz. Die wässrigen Tensid-Zusammensetzungen neigen nicht zu Ausfällungen oder Austrübungen.
- Ausreichend hohe Viskosität, worunter im Rahmen der vorliegenden Erfindung ein Wert von 1000 mPas oder höher verstanden wird (gemessen mit einem Brookfield RV Laborrheometer bei 23°C, 12 U/min, Spindelset RV 02 bis 07 (Spindelauswahl je nach Viskositätbereich)). "mPas" bedeutet bekanntlich Millipascalsekunden
- Gute Reinigungsleistung. Die wässrigen Tensidzusammensetzungen eignen sich, um Anschmutzungen, insbesondere fett- oder ölhaltige Verschmutzungen, von festen oder textilen Oberflächen zu entfernen und zu emulgieren.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform gilt die Maßgabe, dass der Anteil der Verbindungen (A) in den wässrigen Tensid-Zusammensetzungen, bei denen der Rest R¹ ein Alkenylrest ist, - bezogen auf die Gesamtmenge der Verbindungen (A) - bei 3 Gew.-% oder weniger liegt.

In einer bevorzugten Ausführungsform bedeutet der Rest R¹ in der Formel (I) einen gesättigten, linearen Alkylrest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 70 Gew.-% oder mehr und vorzugsweise bei 90 Gew.-% oder mehr liegt.

Vorzugsweise werden die Reste M¹ und M² in der Formel (I) ausgewählt aus der Gruppe H(Wasserstoff) und Na (Natrium).

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind nachfolgend beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.-%,
- der Gehalt an (C) liegt im Bereich von 0 bis 20 Gew.-%,
- der Gehalt an (D) liegt im Bereich von 0 bis 20 Gew.-%,
   mit der Maßgabe, dass die Summe der Komponenten (A), (C) und (D) in dieser Mischung 100 Gew.-% beträgt.

### Zu den Verbindungen (B)

Die Verbindungen (B), die im Rahmen der vorliegenden Erfindung als **Sulfosuccinate** bezeichnet werden, sind für die erfindungsgemäßen wässrigen Tensid-Zusammensetzungen obligatorisch. Sie haben die oben angegebene Formel (II)

R³¹-O-CO-CH(SO₃M¹⁶)-CH₂-COOM¹⁷ (II)

wobei der Rest R³¹ linearen oder verzweigten Alkyl- oder Alkenylrest mit 6 bis 22 C-Atomen oder einen alkoxylierten linearen oder verzweigten Alkyl- oder Alkenylrest mit 6 bis 22 C-Atomen bedeutet und die Reste M¹⁶ und M¹⁷- unabhängig voneinander - ausgewählt werden aus der Gruppe H, Li, Na, K, Ca/2, Mg/2, Ammonium und Alkanolamine.

Die Verbindungen (B) können nach allen dem Fachmann einschlägig bekannten Methoden hergestellt werden. Ein wichtiger Zugang zu den Verbindungen (B) ist der folgende: In einem zweistufigen Prozess wird zunächst Maleinsäureanhydrid mit einem Alkohol oder einem Alkoholalkoxylat, worunter ein Anlagerungsprodukt von Ethylenoxid an einen Alkohol zu verstehen ist, verestert. Der dabei erhaltene Maleinsäureester wird anschließend in wässriger Natriumhydrogensulfit-Lösung sulfoniert.

In einer Ausführungsform gilt die Maßgabe, dass der Anteil der Verbindungen (B) in den wässrigen Tensid-Zusammensetzungen, bei denen der Rest R³¹ ein Alkenylrest ist, - bezogen auf die Gesamtmenge der Verbindungen (B) - bei 3 Gew.-% oder weniger liegt.

In einer Ausführungsform bedeutet der Rest R³¹ in der Formel (II) einen gesättigten, linearen Alkylrest mit 12 bis 18 C-Atomen, wobei in Bezug auf die Verbindungen (B) gilt, dass der Anteil der Verbindungen (B), bei denen der Rest R³¹ ein Dodecyl- und/oder ein Tetradecylrest ist, - bezogen auf die Gesamtmenge der Verbindungen (B) - bei 70 Gew.-% oder mehr und vorzugsweise bei 90 Gew.-% oder mehr liegt.

In einer Ausführungsform bedeutet der Rest R³¹ in der Formel (II) einen linearen Alkylrest mit 8 bis 18 und insbesondere mit 12 bis 18 C-Atomen.

In einer weiteren bevorzugten Ausführungsform bedeutet der Rest R³¹ in der Formel (II) ein Gruppe -(CH₂-CH₂-O)ₚ-R⁵, wobei p eine Zahl im Bereich von 1 bis 4 bedeutet und der Rest R⁵ ein linearer Alkylrest mit 12 bis 18 C-Atomen ist.

Besonders bevorzugt sind Verbindungen (B) mit der INCI-Bezeichnung Disodium Laureth Sulfosuccinate und Disodium Lauryl Sulfosuccinate.

Vorzugsweise werden die Reste M¹⁶ und M¹⁷ in der Formel (II) ausgewählt aus der Gruppe H (Wasserstoff) und Na (Natrium).

Die erfindungsgemäßen wässrigen Tensid-Zusammensetzungen enthalten neben den Verbindungen (A), (B) und Wasser zusätzlich ein oder mehrere **Verbindungen (C)** der allgemeinen Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Ebenfalls enthalten die erfindungsgemäßen wässrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

Dabei ist es besonders bevorzugt, wenn die Reste M¹ und M² der Verbindungen (A), die Reste M¹⁶ und M¹⁷ der Verbindungen (B), der Rest M⁵ der Verbindungen (C) und der Rest M⁶ der Verbindungen (D) ausgewählt werden aus der Gruppe H (Wasserstoff) und Na (Natrium).

In einer Ausführungsform enthalten die erfindungsgemäßen wässrigen Tensid-Zusammensetzungen neben den Verbindungen (A), (B), (C), (D) und Wasser zusätzlich ein oder mehrere **Verbindungen (F)** der allgemeinen Formel (VI)

R⁶CH₂-CO-CHR⁷(SO₃M⁸) (VI),

worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (F) werden im Rahmen der vorliegenden Erfindung als Mono-Sulfo-Ketone bezeichnet.

In einer bevorzugten Ausführungsform bedeuten die Reste R⁶ und R⁷ in der Formel (VI) - unabhängig voneinander - einen gesättigten, linearen Rest mit 10 bis 16 C-Atomen, wobei in Bezug auf die Verbindungen (F) gilt, dass der Anteil der Verbindungen (F), bei denen die Reste R⁶ und R⁷ einen Decyl- und/oder ein Dodecylrest bedeuten, - bezogen auf die Gesamtmenge der Verbindungen (F) - bei 70 Gew.-% oder mehr und vorzugsweise bei 90 Gew.-% oder mehr liegt. Vorzugsweise wird der Rest M⁸ in der Formel (VI) ausgewählt aus der Gruppe H und Na.

In einer Ausführungsform enthalten die erfindungsgemäßen wässrigen Tensid-Zusammensetzungen neben den Verbindungen (A), (B), (C), (D) und Wasser zusätzlich ein oder mehrere **Verbindungen (G)** der allgemeinen Formel (VII)

(SO₃M⁹)R⁸CH-CO-CHR⁹(SO₃M¹⁰) (VII),

worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (G) werden im Rahmen der vorliegenden Erfindung als Di-Sulfo-Ketone bezeichnet.

In einer bevorzugten Ausführungsform bedeuten die Reste R⁸ und R⁹ in der Formel (VII) - unabhängig voneinander - einen gesättigten, linearen Rest mit 10 bis 16 C-Atomen, wobei in Bezug auf die Verbindungen (G) gilt, dass der Anteil der Verbindungen (G), bei denen die Reste R⁸ und R⁹ einen Decyl- und/oder ein Dodecylrest bedeuten, - bezogen auf die Gesamtmenge der Verbindungen (G) - bei 70 Gew.-% oder mehr und vorzugsweise bei 90 Gew.-% oder mehr liegt. Vorzugsweise werden die Reste M⁹ und M¹⁰ in der Formel (VII) ausgewählt aus der Gruppe H und Na.

Die Herstellung der Verbindungen (F) und (G) unterliegt keinen besonderen Einschränkungen und sie können nach allen dem Fachmann bekannten Methoden hergestellt werden.

In einer Ausführungsform werden die Verbindung (F) und (G) durch Sulfonierung entsprechender Ketone mit gasförmigem Schwefeltrioxid hergestellt, wie in der deutschen Offenlegungsschrift DE-A-42,20,580 beschrieben.

In einer anderen Ausführungsform geht man zur Herstellung der Verbindungen (F) und (G) von Fettsäuren aus. Dabei führt man die Sulfierung von flüssigen Fettsäuren mit gasförmigem Schwefeltrioxid so durch, dass dabei neben Disalzen (A) auch die Verbindungen (F) und (G) entstehen, was dadurch realisiert werden kann, dass man die Sulfierung wie folgt durchführt: Das Verhältnis der Rohstoffe Fettsäure, die auch in Form von Gemischen von Fettsäuren unterschiedlicher Kettenlänge eingesetzt werden können, und Schwefeltrioxid wird so eingestellt, dass man 1,0 bis 1,5 mol und insbesondere 1,0 bis 1,25 mol SO₃ pro mol Fettsäure(n) einsetzt. Die Fettsäuren werden dabei mit einer Vorlagetemperatur im Bereich von 70 bis 100 °C in den Reaktor eingebracht. Nach der Sulfierung wird das erhaltene flüssige Sulfierprodukt in einer temperierten Nachreaktionsschlange für 5 bis 20 Minuten auf dieser Temperatur gehalten und gealtert. Anschließend erfolgt die Neutralisation mit einer wässrigen Base, vorzugsweise Natriumhydroxid, in der Regel bei einem pH-Wert im Bereich von von 5 bis 10, insbesondere von 5 bis 7. Im Anschluss kann eine saure Bleiche - der pH wird hierbei auf einen Wert von 7 oder weniger eingestellt - mit Wasserstoffperoxid durchgeführt werden.

In einer Ausführungsform enthalten die erfindungsgemäßen wässrigen Tensid-Zusammensetzungen die Verbindungen (A), (B), (C), (D) und (F). Dabei ist es besonders bevorzugt, wenn die Reste M¹ und M² der Verbindungen (A), die Reste M¹⁶ und M¹⁷ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Menge der Verbindungen (F).

In einer Ausführungsform enthalten die erfindungsgemäßen wässrigen Tensid-Zusammensetzungen die Verbindungen (A), (B), (C), (D) und (G). Dabei ist es besonders bevorzugt, wenn die Reste M¹ und M² der Verbindungen (A), die Reste M¹⁶ und M¹⁷ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Menge der Verbindungen (G).

In einer Ausführungsform enthalten die erfindungsgemäßen wässrigen Tensid-Zusammensetzungen die Verbindungen (A), (B), (C), (D), (F) und (G). Dabei ist es besonders bevorzugt, wenn die Reste M¹ und M² der Verbindungen (A), die Reste M¹⁶ und M¹⁷ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Summe der Menge der Verbindungen (F) und (G).

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A), (B), (D), (E), (F) oder (G) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

### Beispiele

### Eingesetzte Substanzen

### VE-Wasser = vollentsalztes Wasser

**SFA:** alpha-Sulfofettsäuredisalz technischer Qualität auf Basis von nativer C_{12/14}-Fettsäure; Zusammensetzung: 74 Gew.-% Dinatrium-2-Sulfolaurat, 13 Gew.-% Natrium-Laurat, 11 Gew.-% Natriumsulfat, 2 Gew.-% Wasser. Die Bezeichnung "Laurat" bedeutet hierbei, dass das C_{12/14}-Gewichtsverhältnis der Mischung der zu Grunde liegenden nativen Fettsäuren 70 : 30 beträgt.

**SB3:** Texapon SB3, Disodium Laureth Sulfosuccinate (INCI-Bezeichnung), 33 Gew.-% Aktivsubstanz, Handelsprodukt der Fa. BASF PCN

### Mess- und Prüfmethoden

### Bestimmung des Schaumvermögens:

Zur Prüfung des Anschäumverhaltens (Rotorschaum-Methode) wurde ein handelsübliches Messgerät eingesetzt (Sita Foam Tester R-2000). Dabei wurde zunächst eine wäßrige Tensidlösung wie folgt hergestellt: 1 g Aktivsubstanz der jeweils zu prüfenden Probe (als Proben wurden SFA oder SB3 oder Mischungen dieser Substanzen eingesetzt, siehe unten; beim SFA wird - wie oben angegeben - unter Aktivsubstanzgehalt der Disalzgehalt verstanden) wurde bei 20 °C in 1 Liter VE-Wasser gelöst. Der pH-Wert der Lösung wurde mit Citronensäure auf einen Wert von 5,5 eingestellt. Die so hergestellte Lösung wurde temperiert auf 30°C.

Messung: Aus der temperierten Vorlage wurden 250ml in das Messgerät überführt und bei einer Umdrehungszahl von 1300 Umdrehungen pro Minute wurde für 10 Sekunden aufgeschäumt, das dann vorliegende Schaumvolumen (in ml) ermittelt, dann weitere 10 Sekunden geschäumt, das dann vorliegende Schaumvolumen (in ml) ermittelt, usw., d.h. nach jeweils 10 Sekunden währendem Aufschäumen wurde die Schaumhöhe bestimmt. Nach 80 Sekunden Aufschäumzeit wurde die Messung beendet. Die Messung wurde bei jeder Probe 3-mal mit jeweils frischer Lösung aus dem gleichen Ansatz wiederholt und das Ergebnis der Messungen nach 80 Sekunden als Mittelwert aus diesen drei Messungen angegeben (siehe Tabelle).

### Beispiele

### B1 = Beispiel 1 (erfindungsgemäß):

Es wurde eine Mischung von SFA und Texapon SB3 eingesetzt, wobei das Gewichtsverhältnis der jeweiligen Aktivsubstanz von SFA und SB3 auf einen Wert von **2 : 1** eingestellt war. Die Versuchsdurchführung erfolgte wie oben unter "Bestimmung des Schaumvermögens" beschrieben. Die Versuchsdaten können Tabelle 1 entnommen werden.

### B2 = Beispiel 2 (erfindungsgemäß):

Es wurde eine Mischung von SFA und Texapon SB3 eingesetzt, wobei das Gewichtsverhältnis der jeweiligen Aktivsubstanz von SFA und SB3 auf einen Wert von **1 : 1** eingestellt war.

### B3 = Beispiel 3 (erfindungsgemäß):

Es wurde eine Mischung von SFA und Texapon SB3 eingesetzt, wobei das Gewichtsverhältnis der jeweiligen Aktivsubstanz von SFA und SB3 auf einen Wert von **1 : 2** eingestellt war.

### B4 = Beispiel 4 (erfindungsgemäß):

Es wurde eine Mischung von SFA und Texapon SB3 eingesetzt, wobei das Gewichtsverhältnis der jeweiligen Aktivsubstanz von SFA und SB3 auf einen Wert von **1 : 5** eingestellt war.

### V1 = Vergleichsbeispiel 1:

Es wurde ausschließlich SFA eingesetzt.

### V2 = Vergleichsbeispiel 2:

Es wurde ausschließlich SB3 eingesetzt.

**Tabelle 1: Bestimmung des Schaumvermögens**

| | B1 | B2 | B3 | B4 | V1 | V2 |
|---|---|---|---|---|---|---|
| Verhältnis SFA : SB3 | 2:1 | 1:1 | 1:2 | 1:5 | 1:0 | 0:1 |
| Schaumvolumen nach 80 sec | 808 ml | 858 ml | 845 ml | 844 ml | 454 ml | 865 ml |

SFA alleine zeigt ein unbefriedigendes Schaumvolumen (Vergleichsbeispiel 1), während alle Abmischungen von SFA mit SB3 (überraschenderweise auch Beispiel 1 mit einem deutlich SFA Überschuß) ein sehr hohes Schaumvolumen aufweisen, vergleichbar mit dem des SB3.

## Patentansprüche

1. Wässrige Tensid-Zusammensetzungen enthaltend
• ein oder mehrere **alpha-Sulfofettsäuredisalze (A)** der allgemeinen Formel (I),
R¹CH(SO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamin,
• ein oder mehrere **Sulfosuccinate (B)** der allgemeinen Formel (II),
R³¹-O-CO-CH(SO₃M¹⁶)-CH₂-COOM¹⁷ (II)
wobei der Rest R³¹ einen linearen oder verzweigten Alkyl- oder Alkenylrest mit 6 bis 22 C-Atomen oder einen alkoxylierten linearen oder verzweigten Alkyl- oder Alkenylrest mit 6 bis 22 C-Atomen bedeutet und die Reste M¹⁶ und M¹⁷ - unabhängig voneinander - ausgewählt werden aus der Gruppe H, Li, Na, K, Ca/2, Mg/2, Ammonium und Alkanolamine,
• ein oder mehrere **Verbindungen (C)** der allgemeinen Formel (III)
R⁴COOM⁵ (III)
worin der Rest R⁴ einen linearen oder verzweigten Alkyl- oder Alkenylrest mit 7 bis 19 C-Atomen bedeutet und der Rest M⁵ ausgewählt wird aus der Gruppe H, Li, Na, K, Ca/2, Mg/2, Ammonium und Alkanolamin,
• ein oder mehrere **anorganische Salze der Schwefelsäure (D)** der allgemeinen Formel (IV)
(M⁶)₂SO₄ (IV)
enthalten, wobei M⁶ ausgewählt wird aus der Gruppe Li, Na, K, Ca/2, Mg/2, Ammonium und Alkanolamin,
• **Wasser,**
wobei folgende Maßgabe gilt:
• Sofern die wässrigen Tensid-Zusammensetzungen ein oder mehrere **Estersulfonate (E)** der allgemeinen Formel (V),
R²CH(SO₃M⁷)COOR³ (V)
worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 90 Gew.-% oder mehr vorliegen müssen.

2. Zusammensetzungen nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Zusammensetzungen nach Anspruch 1 oder 2, wobei die Reste M¹ und M² ausgewählt werden aus der Gruppe H (Wasserstoff) und Na (Natrium).

4. Zusammensetzungen nach einem der Ansprüche 1 bis 3, wobei die Zusammensetzungen zusätzlich ein oder mehrere **Mono-Sulfo-Ketone (F)** der allgemeinen Formel (VI)
R⁶CH₂-CO-CHR⁷(SO₃M⁸) (VI),
worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine.

5. Zusammensetzungen nach einem der Ansprüche 1 bis 4, wobei die Zusammensetzungen zusätzlich ein oder mehrere **Di-Sulfo-Ketone (G)** der allgemeinen Formel (VII)
(SO₃M⁹)R⁸CH-CO-CHR⁹(SO₃M¹⁰) (VII),
worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine.

6. Verwendung der Zusammensetzungen nach einem der Ansprüche 1 bis 5 für kosmetische Mittel sowie Wasch- und Reinigungsmittel.

7. Verwendung der Zusammensetzungen nach einem der Ansprüche 1 bis 5 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

8. Verwendung der Zusammensetzungen nach einem der Ansprüche 1 bis 5 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.

## Claims

1. An aqueous surfactant composition comprising
• one or more **alpha-sulfo fatty acid disalts (A)** of general formula (I),
R¹CH(SO₃M¹)COOM² (I)
in which the radical R¹ is a linear or branched alkyl or alkenyl radical having 6 to 18 carbon atoms and the radicals M¹ and M² - independently of each other - are selected from the group comprising H, Li, Na, K, Ca/2, Mg/2, ammonium and alkanolamine,
• one or more **sulfosuccinates (B)** of general formula (II),
R³¹-O-CO-CH(SO₃M¹⁶)-CH₂-COOM¹⁷ (II)
in which the radical R³¹ is a linear or branched alkyl or alkenyl radical having 6 to 22 carbon atoms or an alkoxylated linear or branched alkyl or alkenyl radical having 6 to 22 carbon atoms and the radicals M¹⁶ and M¹⁷ - independently of each other - are selected from the group comprising H, Li, Na, K, Ca/2, Mg/2, ammonium and alkanolamines,
• one or more **compounds (C)** of general formula (III)
R⁴COOM⁵ (III)
in which the radical R⁴ is a linear or branched alkyl or alkenyl radical having 7 to 19 carbon atoms and the radical M⁵ is selected from the group comprising H, Li, Na, K, Ca/2, Mg/2, ammonium and alkanolamine,
• comprise one or more **inorganic salts of sulfuric acid (D)** of general formula (IV)
(M⁶)₂SO₄ (IV)
in which M⁶ is selected from the group comprising Li, Na, K, Ca/2, Mg/2, ammonium and alkanolamine,
• **water,**
where the following proviso applies:
• if the aqueous surfactant compositions comprises one or more **ester sulfonates (E)** of general formula (V),
R²CH(SO₃M⁷)COOR₃ (V)
in which the radical R² is a linear or branched alkyl or alkenyl radical having 6 to 18 carbon atoms and the radical R³ is a linear or branched alkyl or alkenyl radical having 1 to 20 carbon atoms, where the radical R³ can logically be an alkenyl radical or be branched only above 3 carbon atoms, and the radical M⁷ is selected from the group comprising Li, Na, K, Ca/2, Mg/2, ammonium and alkanolamines, it is the case that the compounds (A) - based on the totality of the compounds (A) and (E) - must be present to an extent of 90% by weight or more.

2. The composition according to claim 1, wherein the radical R¹ in the formula (I) is a saturated, linear alkyl radical having 10 to 16 carbon atoms, where with regard to the compounds (A) it is the case that the proportion of the compounds (A) in which the radical R¹ is a decyl or a dodecyl radical - based on the total amount of the compounds (A) - is 90% by weight or more.

3. The composition according to claim 1 or 2, wherein the radicals M¹ and M² are selected from the group comprising H (hydrogen) and Na (sodium).

4. The composition according to any of claims 1 to 3, wherein the composition additionally one or more **monosulfoketones (F)** of general formula (VI)
R⁶CH₂-CO-CHR⁷(SO₃M⁸) (VI),
in which the radicals R⁶ and R⁷ - independently of each other - are a linear or branched alkyl radical having 6 to 18 carbon atoms and the radical M⁸ is selected from the group comprising H, Li, Na, K, Ca/2, Mg/2, ammonium and alkanolamines.

5. The composition according to any of claims 1 to 4, wherein the composition additionally one or more **disulfoketones (G)** of general formula (VII)
(SO₃M⁹)R⁸CH-CO-CHR⁹(SO₃M¹⁰) (VII),
in which the radicals R⁸ and R⁹ - independently of each other - are a linear or branched alkyl radical having 6 to 18 carbon atoms and the radicals M⁹ and M¹⁰ - independently of each other - are selected from the group comprising H, Li, Na, K, Ca/2, Mg/2, ammonium and alkanolamines.

6. The use of the composition according to any of claims 1 to 5 for cosmetic products and also detergents and cleaners.

7. The use of the composition according to any of claims 1 to 5 for cosmetic products in the form of hair shampoos, shower gels, soaps, syndets, washing pastes, washing lotions, scrub preparations, foam baths, oil baths, shower baths, shaving foams, shaving lotions, shaving creams and dental care products.

8. The use of the composition according to any of claims 1 to 5 for products with a low pH for cleaning hard surfaces, such as bath and toilet cleaners and the like, and also for cleaning and/or fragrance gels for use in sanitary installations.

## Revendications

1. Compositions aqueuses de tensioactif contenant
• un ou plusieurs disels d'acide gras alpha-sulfonique (A) de formule générale (I),
**R¹CH(SO₃M¹)COOM²** **(I)**
dans laquelle le radical R¹ signifie un radical alkyle ou alcényle linéaire ou ramifié comportant 6 à 18 atomes de C et les radicaux M¹ et M² - indépendamment l'un de l'autre- sont choisis dans le groupe H, Li, Na, K, Ca/2, Mg/2, ammonium et alcanolamine,
• un ou plusieurs sulfosuccinates (B) de formule générale (II),
**R³¹-O-CO-CH(SO₃M¹⁶)-CH₂-COOM¹⁷** **(ii)**
le radical R³¹ signifiant un radical alkyle ou alcényle linéaire ou ramifié comportant 6 à 22 atomes de C ou un radical alkyle ou alcényle linéaire ou ramifié, alcoxylé, comportant 6 à 22 atomes de C et les radicaux M¹⁶ et M¹⁷ - indépendamment l'un de l'autre - étant choisis dans le groupe H, Li, Na, K, Ca/2, Mg/2, ammonium et alcanolamines,
• un ou plusieurs composés (C) de formule générale (III)
**R⁴COOM⁵** **(III)**
dans laquelle le radical R⁴ signifie un radical alkyle ou alcényle linéaire ou ramifié comportant 7 à 19 atomes de C et le radical M⁵ est choisi dans le groupe H, Li, Na, K, Ca/2, Mg/2, ammonium et alcanolamine,
• contiennent un ou plusieurs sels inorganiques de l'acide sulfurique (D) de formule générale (IV)
**(M⁶)₂SO₄** **(IV)**
M⁶ étant choisi dans le groupe Li, Na, K, Ca/2, Mg/2, ammonium et alcanolamine,
• de l'eau
à condition que :
• si les compositions aqueuses de tensioactif contient un ou plusieurs estersulfonates (E) de formule générale (V),
**R²CH(SO₃M⁷)COOR³** **(V)**
dans laquelle le radical R² signifie un radical alkyle ou alcényle linéaire ou ramifié comportant 6 à 18 atomes de C et le radical R³ signifie un radical alkyle ou alcényle linéaire ou ramifié comportant 1 à 20 atomes de C, le radical R³ pouvant logiquement être un radical alcényle ou être ramifié seulement à partir de 3 atomes de C, et le radical M⁷ étant choisi dans le groupe Li, Na, K, Ca/2, Mg/2, ammonium et alcanolamines, alors les composés (A) - par rapport à la totalité des composés (A) et (E) - doivent être présents à raison de 90 % en poids ou plus.

2. Compositions selon la revendication 1, le radical R¹ dans la formule (I) signifiant un radical alkyle saturé, linéaire comportant 10 à 16 atomes de C, où par rapport aux composés (A), la proportion de composés (A) dans lesquels le radical R¹ est un radical décyle ou dodécyle, par rapport à la quantité totale de composés (A), se situe à 90 % en poids ou plus.

3. Compositions selon la revendication 1 ou 2, les radicaux M¹ et M² étant choisis dans le groupe de H (hydrogène) et Na (sodium).

4. Compositions selon l'une quelconque des revendications 1 à 3, les compositions de plus une ou plusieurs monosulfocétones (F) de formule générale (VI)
**R⁶CH₂-CO-CHR⁷(SO₃M⁸)** **(VI),**
dans laquelle les radicaux R⁶ et R⁷ - indépendamment l'un de l'autre - signifient un radical alkyle linéaire ou ramifié comportant 6 à 18 atomes de C et le radical M⁸ est choisi dans le groupe H, Li, Na, K, Ca/2, Mg/2, ammonium et alcanolamines.

5. Compositions selon l'une quelconque des revendications 1 à 4, les compositions de plus une ou plusieurs disulfocétones (G) de formule générale (VII)
**(SO₃M⁹)R⁸CH-CO-CHR⁹(SO₃M¹⁰)** **(VII),**
dans laquelle les radicaux R⁸ et R⁹ - indépendamment l'un de l'autre - signifient un radical alkyle linéaire ou ramifié comportant 6 à 18 atomes de C et les radicaux M⁹ et M¹⁰ - indépendamment l'un de l'autre - sont choisis dans le groupe H, Li, Na, K, Ca/2, Mg/2, ammonium et alcanolamines.

6. Utilisation des compositions selon l'une quelconque des revendications 1 à 5 pour des produits cosmétiques ainsi que des produits de lavage et de nettoyage.

7. Utilisation des compositions selon l'une quelconque des revendications 1 à 5 pour des produits cosmétiques sous forme de shampooings capillaires, de gels douches, de savons, de syndets, de pâtes de lavage, de lotions de lavage, de préparations de gommage, de bains moussants, de bains d'huile, de bains douches, de mousses à raser, de lotions de rasage, de crèmes à raser et de produits de soins dentaires.

8. Utilisation des compositions selon l'une quelconque des revendications 1 à 5 pour des produits dotés d'une valeur de pH basse pour le nettoyage de surfaces dures, comme des nettoyants de salles de bains et de WC et similaires, ainsi que pour des gels de nettoyage et/ou parfumés pour une application dans des installations sanitaires.
